(19) Europäisches Patentamt
European Patent Office
Office européen des brevets

(11) Publication number: 0 337 585 A1

# (12) EUROPEAN PATENT APPLICATION

(21) Application number: 89201440.8

(22) Date of filing: 02.08.88

(51) Int. Cl.4: C07D 209/12 , C07D 491/04 , //(C07D491/04,311:00,209:00)

(30) Priority: 27.08.87 US 89881

(43) Date of publication of application: 18.10.89 Bulletin 89/42

(60) Publication number of the earlier application in accordance with Art.76 EPC: 0 306 149

(84) Designated Contracting States: AT BE CH DE ES FR GR IT LI LU NL SE

(71) Applicant: AMERICAN HOME PRODUCTS CORPORATION
685, Third Avenue
New York, New York 10017(US)

(72) Inventor: Shaw, Chia-Cheng
2055 Bonin
St Laurent Quebec H4M 1A1(CA)
Inventor: Pelz, Karel
315 St Aubin
St Laurent Quebec H4M 2J1(CA)

(74) Representative: Brown, Keith John Symons et al
c/o John Wyeth & Brother Limited Patent and Trademark Department Huntercombe Lane South
Taplow Maidenhead Berkshire SL6 0PH.(GB)

## (54) Substituted 1,3,4,9-tetrahydropyrano[3,4-b]indole-l-acetic acids.

(57) A novel process is described for the production of substituted 1,3,4,9-tetrahydropyrano[3,4-b]indole-1-acetic acids having useful analgesic and anti-inflammatory activity and having the formula (I)

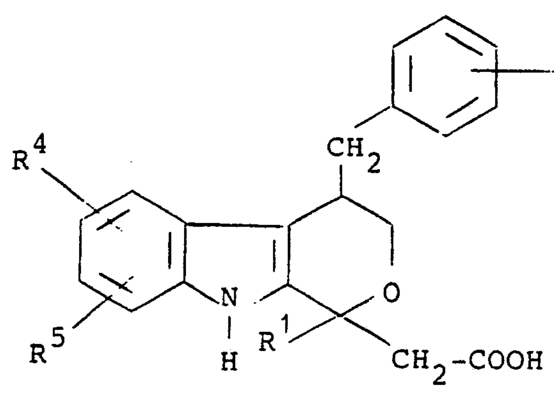

(I)

wherein $R^1$ is lower alkyl, $R^4$ and $R^5$ are hydrogen, lower alkyl or halogen and $R^2$ is hydrogen, 4-halogen 2,4 dihalo, 3-trifluoromethyl or 4-methoxy via the intermediate of formula

EP 0 337 585 A1

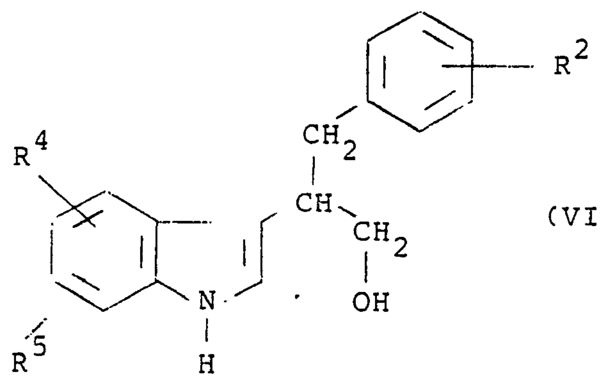

(VII)

which is prepared from an isatin derivative.

## SUBSTITUTED 1,3,4,9-TETRAHYDROPYRANO[3,4-b]INDOLE-1-ACETIC ACIDS

This invention relates to a novel process for the preparation of indole derivatives.

More specifically, this invention relates to the process for the production of tricyclic acetic acid derivaties in which the tricyclic portion thereof is characterised by having an indole portion fused to a pyrano ring. Still more specifically, the process of this invention produces the following tricyclic acetic acid system:

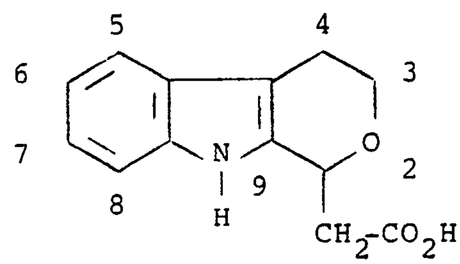

designated 1,3,4,9-tetrahydropyrano[3,4b]indole-1-acetic acid in which the carbons at the 1- and 4-position, and optionally at the 5 , 6-, and 8-positions are further substituted.

The indole derivatives produced by the present process are known to exhibit useful pharmacodynamic properties without eliciting undesireable side effects. Notable attributes of these derivatives are anti-inflammatory and analgesic activities.

The indole derivatives produced by the present process are described in US Patent 4,670,462 and in co-pending European Patent Application 0238226, Demerson et al, United States Patent No 3939178 discloses the production of 1,3,4,9-tetrahydrothiopyrano[3,4-b]-indoles and 1,3,4,9-tetrahydropyrano[3,4-b]-indoles having analgesic and anti-inflammatory activity. Related United States Patents are Nos 3974179 and 3,843,681.

The process of the present invention is directed to the production of the compounds represented by formula (I)

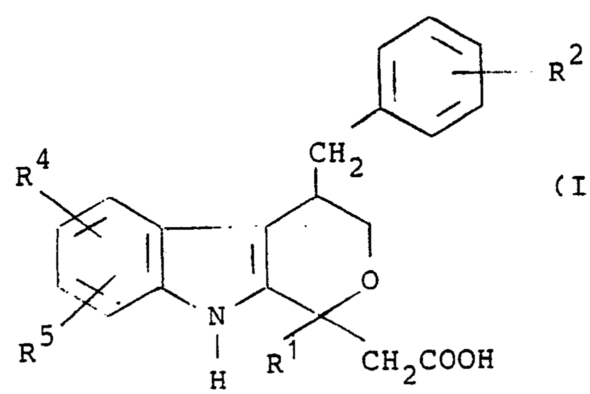

(I)

wherein $R^1$ is lower alkyl containing 1 to 4 carbon atoms; $R^4$ and $R^5$ are hydrogen, lower alkyl containing 1 to 6 carbon atoms, or halogen; and $R^2$ is hydrogen, 4-halogen, 2- and 4-dihalogen, 3-trifluoromethyl, or 4-methoxy; and the pharmaceutically acceptable salts thereof.

The preferred process of the present invention is directed to the production of the compound designated 1-ethyl 1,3,4,9-tetrahydro-4-(phenylmethyl)pyrano[3,4-b]indole-1-acetic acid, and the pharmaceutically acceptable salts thereof.

A still further preferred aspect of the present invention is directed to the production of cis-1-ethyl-1,3,4,9-tetrahydro-4-(phenylmethyl) pyrano[3,4-b]indole-1-acetic acid, and the pharmaceuically acceptable salts thereof.

The products of formula (I) may be prepared via an intermediate β-benzyltryptophol of general formula (VII)

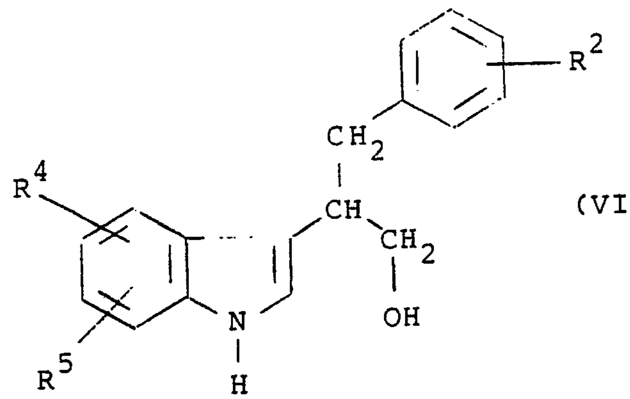

(VII)

where $R^2$, $R^4$ and $R^5$ have the meanings given above.

According to the present invenion the β-benzyltryptophol is prepared by a process which comprises

(a) alkylating an isatin of formula (XI)

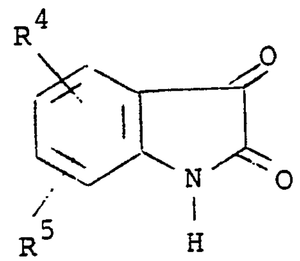

(XI)

where $R^4$ and $R^5$ are as defined above with an alkyl 3-phenylpropionate of formula

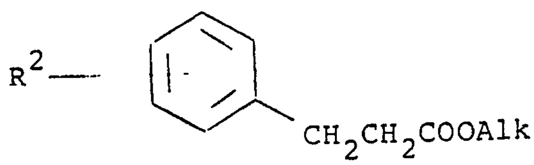

where $R^2$ is as defined above and Alk is lower alkyl containing 1 to 6 carbon atoms to produce a compound of formula (XIII)

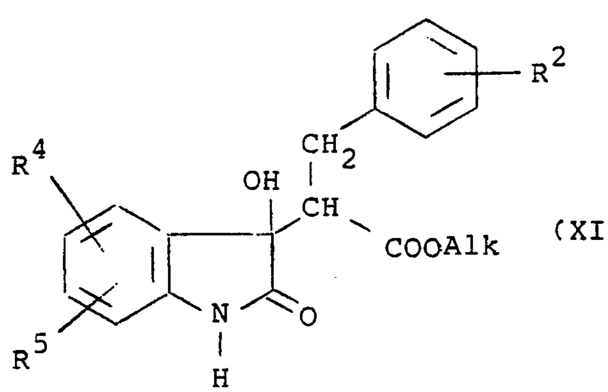

(XIII)

where $R^2$, $R^4$, $R^5$ and Alk are as defined above

and

(b) reducing said compound of formula (XIII) with sodium borohydride and boronitrifluoride etherate to produce the substituted β-benzyltryptophol of formula (VII).

The above mentioned process and the conversion of the β-benzyltryptophol into the product of formula (I) are represented, by way of example, in the following flow sheet.

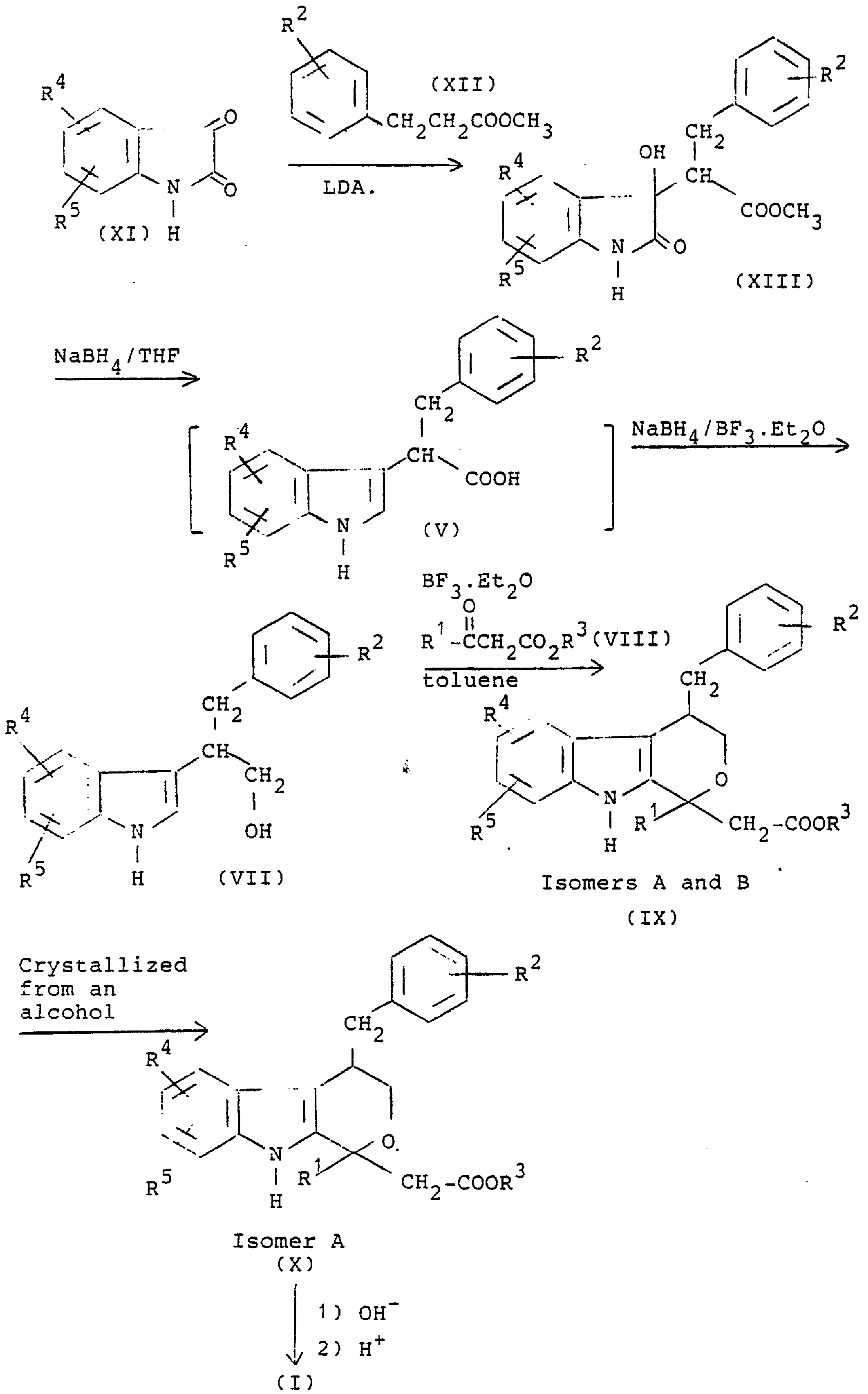
(XI)
(XII)
LDA.
(XIII)
NaBH4/THF
(V)
NaBH4/BF3.Et2O
BF3.Et2O
(VIII)
toluene
(VII)
Isomers A and B
(IX)
Crystallized from an alcohol
Isomer A
(X)
1) OH-
2) H+
(I)

wherein $R^1$, $R^2$, $R^4$, and $R^5$ are as defined above; and $R^3$ is lower alkyl containing 1 to 8 carbon atoms.

Referring to the flow sheet, in the reaction of isatin (XI) with methyl 3-phenylpropionate (XII), good yields were obtained by reversed quenching at slightly acidic pH, ie addition of the reaction mixture to an aqueous solution containing acetic acid in order to minimize reverse aldolization of alkaline aqueous medium.

For the reduction of compound (XIII) to tryptophol (VII), the safe and inexpensive sodium borohydride and borontrifluoride etherate regents were used. This reduction proceeds in two stages. First, compound (XIII) is readily and quantatively reduced and simultaneously the ester is hydrolyzed by $NaBH_4$ in dry THF to give the benzylindoylacetic acid (V). Subsequent addition of $BF_3.Et_2O$ completes in about two hours the reduction of the carboxylic group to the primary alcohol, and $\beta$-benzyltryptophol (VII) is obtained in a good yield of 57.0% overall from isatin.

For comparison, reduction with lithium aluminium hydride produced tryptophol (VII) in variable yields ranging from 25% to 49% overall from isatin.

It should be noted that direct reduction of ester (XIII) with diborane requires about 15 hours at reflux for completion and the yields are inferior to the above two-stage method.

Production of the ester (IX) was preferably carried out by reaction of the substituted $\beta$-benzyltryptophol (VII) with 3-oxo-2-alkanoic acid, alkyl ester of formula (VIII) at about -15° C in toluene.

Production of the ester (XI) was preferably done by a reversed addition of the substituted $\beta$-benzyltryptophol (VII) in toluene solution to a cold mixture of the 3-oxo-2-alkanoic acid, alkyl ester (VIII) and boron trifluoride etherate in toluene. The advantage of the reversed addition is that the product (IX) isolated had a more constant ratio of the two isomers A and B. The ratio of the two diastereomers A and B is about 2:1 in favour of the desirable isomer A.

The starting material (VII) and the product (IX) are not stable in solution, in the presence of boron trifluoride etherate, thus, prolonged reaction times at higher temperatures should be avoided to prevent formation of polar impurities. The crude product (IX), without filtration through silica gel, was directly fractionally crystallized to obtain the pure crystalline product (X) isomer A.

The product (X) isomer A was subjected to basic hydrolysis and upon acidification the product (I) was obtained in high yield. The final purification of (I) was carried out by recrystallization from aqueous alcohols preferably from ethanol/water or isopropanol/water.

The compounds of formula (I) form salts with suitable pharmaceutically acceptable inorganic and organic bases. The acid of formula (I) is transformed in excellent yield into the corresponding pharmaceutically acceptable salts by neutralization of said acid with the appropriate inorganic or organic base. Suitable inorganic bases to form these salts include, for example, the hydroxides, carbonates, bicarbonates, or alkoxides of the alkali metals, or alkaline earth metals, for example, sodium, potassium, magnesium, calcium, and the like. Suitable organic bases include the following amines; lower mono-, di- and tri-alkylamines, the alkyl radicals of which contain up to three carbon atoms, such as methylamine, dimethylamine, trimethylamine, ethylamine, di- and triethylamine, methyethylamine, and the like; mono, di- and trialkanolamines, the alkanol radicals of which contain up to three carbon atoms, such as mono-, di-and triethanolamine; alkylenediamines which contain up to six carbon atoms, such as hexamethyleneamine; amino sugars, such as glucosamine; cyclic saturated or unsaturated bases containing up to six carbon atoms, such as pyrrolidine, piperidine, morpholine, piperazine, and their N-alkyl and N-hydroxyalkyl derivatives, such as N-methylmorpholine and N-(2-hydroxyethyl)piperidine, as well as pyridine. Furthermore, there may be mentioned the corresponding quaternary salts, such as the tetraalkyl (for example tetramethyl), alkyl-alkanol (for example methyltrimethanol, and trimethyl-monoethanol) and cyclic ammonium salts, for example the N-dimethylpyridinium, N-methyl-N-(2-hydroxyethyl)-morpholinium, N,N-dimethylmorpholinium, N-methyl-N-(2-hydroxyethyl)-morpholinium, N,N-dimethylpiperidinium salts, which are characterized by good water-solubility.

The transformations to the salts can be carried out by a variety of methods known in the art. For example, in the case of salts of inorganic bases, it is preferred to dissolve the acid of formula (I) in water containing at least one equivalent amount of a hydroxide, carbonate, or bicarbonate. Preferably, the reaction is performed in a water-miscible organic solvent inert to the reaction conditions, for example, methanol, ethanol, dioxane, and the like in the presence of water. For example, such use of sodium hydroxide, sodium carbonate, or sodium bicarbonate gives a solution of the sodium salts. Evaporation of the solution or addition of a water-miscible solvent of a more moderate polarity, for example, a lower alkanol, for instance, butanol, or a lower alkanone, for example, ethyl methyl ketone, gives the solid salt if that form is desired.

To produce an amine salt, the acid of formula (I) is dissolved in a suitable solvent of either moderate or low polarity, for example, ethanol, acetone, ethyl acetate, diethyl ether, or benzene. At least an equivalent amount of the amine corresponding to the desired cation is then added to that solution. If the resulting salt

does not precipitate, it can usually be obtained in solid form by addition of a miscible diluent of low polarity, for example, benzene or petroleum ether, or by evaporation. If the amine is relatively volatile, any excess can easily be removed by evaporation. It is preferred to use substantially equivalent amounts of the less volatile amines.

Salts wherein the cation is quaternary ammonium are produced by mixing the acid of formula (I) with an equivalent amount of the corresponding quaternary ammonium hydroxide in water solution, followed by evaporation of the water.

Included in the present invention is the process for the production of diastereoisomers of compound (I) wherein the 4-substituent is either cis or trans to the acetic acid chain at position one. When the structure of the diastereomers is not known, they are designated Isomer A and B.

Also included in this invention is the process for the resolution of the compounds of formula (I) into their optical isomers. The optical isomers of the compounds of formula (I) result from asymmetric centres, contained therein. Such isomers can be obtained in substantially pure form by classical separation techniques and by sterically controlled synthesis. Included is the specific case of the resolution of 1-ethyl-1,3,4,9-tetrahydro-4-(phenylmethyl)-pyrano[3,4-b]indole-1-acetic acid into its optical isomers by separation of the corresponding [(1S)endo]1,7,7-trimethyl-bicyclo[2.2.1]heptan-2-yl ester followed by basic hydrolysis.

The following examples further illustrate the present invention.

## EXAMPLE 1

### Preparation of Methyl 2,3-Dihydro-3-hydroxy-α-(phenylmethyl)-2-oxo-1H-indole-3-acetate

To a cooled (-5° C) suspension of isatin (8.85 g, 0.0602 mol), and methyl 3-phenylpropionate (9.88 g, 0.0602 mol) in 220 mL of dry tetrahydrofuran under a nitrogen atmosphere was added lithium diisopropylamide (LDA) (75 mL, 0.1275 mol of a 1.72 M solution) over a 1½ hour period. The reaction mixture was stirred at -5° C for one hour and then poured into a cooled (0 to 5° C) solution of toluene (140 mL), and acetic acid (30.6 g, 0.51 mol, 300% excess) over a ½ hour period under a nitrogen atmosphere. The transfer was completed with a 200 mL water wash.

The mixture was stirred for ½ hour at 5 to 10° C at pH 5.5. The aqueous layer was separated and extracted with toluene (140 mL). The combined toluene phases were washed with 10% brine (50 mL) and stirred for ½ hour with activated charcoal (4.4 g). The charcoal was removed by filtration and washed with toluene (2 x 50 mL). The filtrate was evaporated to dryness in vacuo to give 11.82 g of residue containing 7.48 g of product (yield 68.0%).

## EXAMPLE 2

### Preparation of β-Benzyltryptophol

(VII: $R^2$, $R^4$, $R^5$ = -H)

To a solutiuon of methyl 2,3-dihydro-3-hydroxy-α-(phenylmethyl)-2-oxo-1H indole-3-acetate (3.11 g, 10 mmol) in tetrahydrofuran (40 mL) under a nitrogen atmosphere was added in portions at 25° C, sodium borohydride (0.76 g, 20 mmol). The mixture was stirred at 25° C for one hour. To this mixture was added, over half an hour at 25° C, borontrifluoride-etherate (3.8 g, 26.6 mmol). The mixture was stirred at 25° C for 1.5 hours, then it was refluxed for 2 hours, then cooled to 20° C.

To the reaction mixture was added 50 mL toluene. followed by the slow addition of 50 mL 2N HCl, then it was stirred at 25° C for half an hour. The organic phase was separated and washed with 2 x 25 mL water, then it was evaporated on the rotovap to dryness to give a residue of 2.82 g containing 2.13 g of β-benzyltryptophol, yield 84.8%.

## EXAMPLE 3

### Preparation of β-Benzyltryptophol

(VII: $R^2$, $R^4$, $R^5$ = -H)

To a solution of crude methyl 2,3-dihydro-3-hydroxy-α-(phenylmethyl)-2-oxo-1H indole-3-acetate (9.05 g obtained from 4.43 g isatin by the process of Example 1) in tetrahydrofuran (63 mL) under a nitrogen atmosphere was added in portions at 25° C, sodium borohydride (1.79 g). The mixture was stirred at 25° C overnight. To this mixture was added, over half an hour at 20° C, borontrifluoride-etherate (8.91 g). The mixture was stirred at 20° C for 2 hours, and then 2 hours at 50° C, then cooled to 20° C.

To the reaction mixture was added 80 mL toluene, followed by the slow addition of 32 mL $H_2O$ and 3 mL 2N HCl to pH2. The organic phase was separated and washed with 2 x 25 mL water, then it was evaporated on the rotovap to dryness to give a residue containing 4.3 g of β-benzyltryptophol, yield 57.0% overall from isatin.

## EXAMPLE 4

### Preparation of cis-1-Ethyl-1,3,4,9-tetrahydro-4-(phenylmethyl) pyrano[3,4-b]indole-1-acetic Acid Methyl Ester

(X: $R^2$, $R^4$, $R^5$ = -H; $R^3$ = $-CH_3$)

To a solution of boron trifluoride etherate (10.9 g) in dry toluene (45 mL), cooled to 0° C was added 5.0 g of methyl propionyl acetate. The mixture was stirred and cooled to -15° ±2° C under nitrogen.

To the above cold solution was added dropwise, over a period of 30 minutes, a solution of β-benzyltryptophol (9.62 g) in dry toluene (30 mL) while maintaining the pot temperature at -15° to -10° C. After the addition, the mixture was stirred at -15° ±2° C for 4 hours, then left in the cold room (-15° C) without stirring overnight.

To the cold reaction mixture was added 9 mL of pyridine over a period of 15 minutes while maintaining the pot temperature below +10° C (a mass of organic salt formed). After the addition, the mixture was stirred for 10 minutes, then 30 mL of cold water was added, and the mixture stirred vigorously for 10 minutes. The aqueous phase was separated and extracted with 40 mL of toluene. The toluene extracts were combined, washed with water (50 mL x 2), then 10% hydrochloric acid (40 mL), followed by water (50 mL x 3).

The organic phase was filtered and evaporated under reduced pressure to give 13.46 g of crude product as a beige-coloured gum.

A quantitative HPLC analysis of this product showed it contained 53.0% of the cis-diastereomer and 25.0% of the trans-diastereomer (ratio of two diastereomers was 2.1:1). The crude product slowly crystallized on standing at room temperature.

The combined yield of both isomers was 76.4%.

The cis-diastereomer was obtained by crystallization from isopropanol giving 82.3% recovery, mp 119.5-120.5° C. HPLC showed 96.6% cis-isomer and 1.7% trans-isomer.

## EXAMPLE 5

### Preparation of cis-1-Ethyl-1,3,4,9-tetrahydro 4-(phenylmethyl)-pyrano[3,4-b]indole-1-acetic Acid

(I: $R^1$ = $C_2H_5$; $R^2$, $R^4$, $R^5$ = -H)

cis-1-Ethyl-1,3,4,9-tetrahydro-4-(phenylmethyl)-pyrano[3,4-b]indole-1-acetic acid methyl ester in 3 parts methanol and aqueous NaOH was refluxed for 45 minutes. The methanol was stripped off and replaced with water/EtOH for a final concentration of 6 parts water and 3 parts EtOH. Acetic acid was added (20% excess based on NaOH) at 50° C and the crude product was allowed to crystallize at 50° C. Then the pH was adjusted to 2 at 25° C with hydrochloric acid/water (1:1).

The crude product was filtered and dried at 60° C for 20 hours to give 95.0% yield, mp 145.5-147° (dec). HPLC showed 98.6% purity.

The crude product was recrystallized from isopropanol/water (2:1) to give 99.7% pure product, mp 147-148° C.

## Claims

1. A process for preparing a substituted β-benzyltryptophol of general formula (VII)

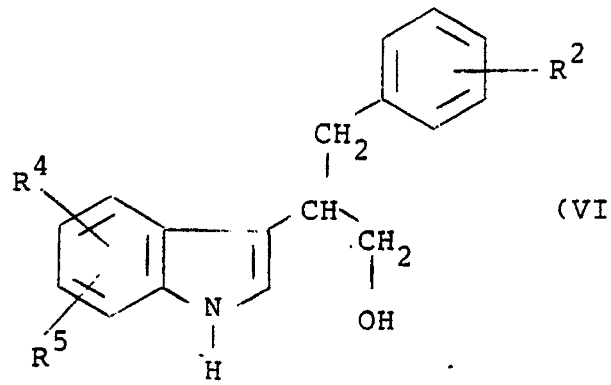

(VII)

wherein $R^4$ and $R^5$ are hydrogen, lower alkyl containing 1 to 6 carbon atoms, or halogen; and $R^2$ is hydrogen,4-halogen, 2- and 4-dihalogen, 3-trifluoromethyl or 4-methoxy which comprises

(a) alkylating an isatin of formula (XI)

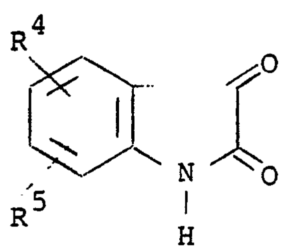

(XI)

where $R^4$ and $R^5$ are as defined above with an alkyl 3-phenylpropionate of formula

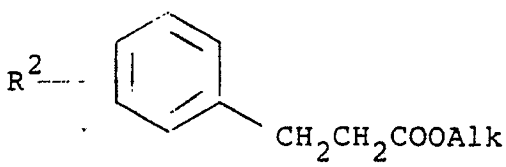

where $R^2$ is as defined above and Alk is lower alkyl containing 1 to 6 carbon atoms to produce a compound of Formula XIII

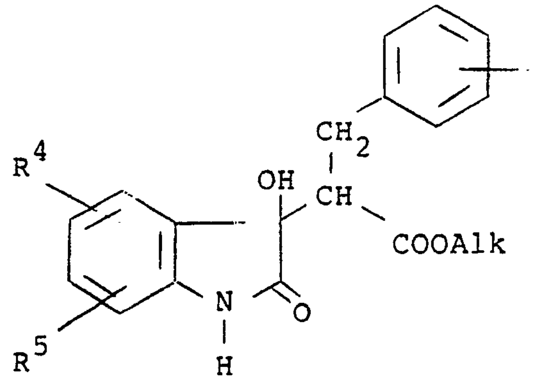

(XIII)

where $R^2$, $R^4$, $R^5$ and Alk are as defined above and

(b) reducing said compound of formula (XIII) with sodium borohydride and boronitrifluoride etherate to produce the substituted β-benzyltryptophol of formula (VII)

2. A process according to claim 1 characterised in that in step (a) the reaction mixture of the isatin (XI) and the alkyl 3-phenylpropionate is subjected to reverse quenching at slightly acidic pH.

3. A process as claimed in claim 1 or 2 in which the tryptophol of formula (VII) is converted into a compound of formula (I)

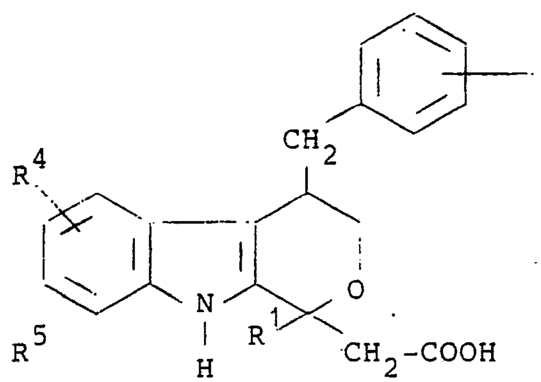

(I)

wherein $R^1$ is lower alkyl containing 1 to 4 carbon atoms and $R^2$, $R^4$ and $R^5$ are as defined in claim 1 or a pharmaceutically acceptable salt thereof by

(i) reacting the tryptophol of formula (VII) with 3-oxo-2-alkanoic acid, alkyl ester of formula (VIII)

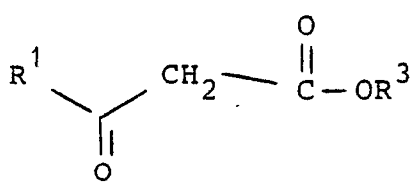

(VIII)

wherein $R^1$ is as defined above and $R^3$ is lower alkyl containing 1 to 8 carbon atoms to produce an alkyl ester of formula (IX)

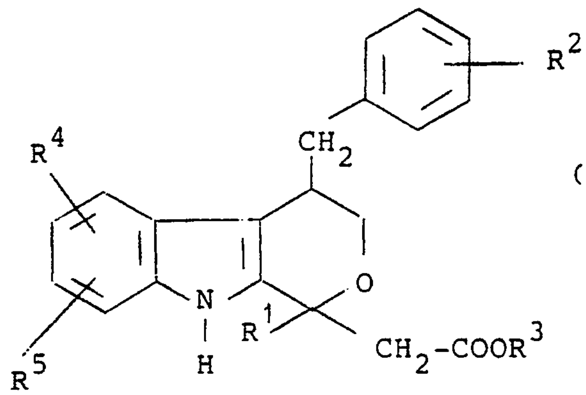

(IX)

wherein $R^1$, $R^2$, $R^3$, $R^4$ and $R^5$ are as defined above and

(ii) hydrolysing the alkyl ester (IX) to obtain the compound of formula (I).

4. A process as claimed in claim 3 wherein the alkyl ester of formula (IX) is obtained as a mixture of isomers, isomer A is separated from isomer B by crystallising it from an alcohol solvent and isomer A is hydrolysed to the desired compound of formula (I).

5. A process as claimed in claim 4 wherein the alcohol is isopropyl alcohol.

6. A process as claimed in claim 3 wherein in step (i) the 3-oxo-2-alkanoic acid, alkyl ester is methyl propionyl acetate and the reaction is carried out in the presence of boron trifluoride etherate.

7. A process as claimed in claim 3 wherein in step (i) a toluene solution of the tryptophol of formula (VII) is added to a cold mixture of the 3-oxo-2-alkanoic acid alkyl ester in boron trifluoride etherate in toluene.

8. A process as claimed in any one of claims 3 to 7 wherein $R^1$ is ethyl and $R^2$, $R^4$ and $R^5$ are hydrogen.

European Patent Office

# EUROPEAN SEARCH REPORT

Application Number

EP 89 20 1440

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | US-A-3 974 179 (C.A. DEMERSON) * column 7, lines 63-68, column 8, lines 1-14, column 9, lines 1-65; column 10, lines 1-45 * | 1,3 | C 07 D 209/12<br>C 07 D 491/04 //<br>(C 07 D 491/04<br>C 07 D 311:00<br>C 07 D 209:00 ) |
| Y,D | US-A-4 670 462 (A.H. KATZ et al.) * column 3, lines 5-45 * | 1,3 | |
| Y | JOURNAL OF MEDICINAL CHEMISTRY vol. 19, no. 3, 1976, pages 391-395; C.A. DEMERSON et al.:"Etodolic Acid and Related Compounds. Chemistry and Antiinflammatory Actions of Some Potent Di- and Trisubstituted 1,3,4,9-Tetrahydropyrano[3,4-b]indole-1-acetic Acids" * page 392, scheme III * | 1,3 | |
| Y | M. FIESER:"REAGENTS FOR ORGANIC SYNTHESIS" vol. 4, 1974, Wiley-Interscience, New York * page 301, paragraphs 3,4 * | 1 | **TECHNICAL FIELDS SEARCHED (Int. Cl.4)** |
| A | CHEMICAL REVIEWS vol. 76, no. 6, December 1976, pages 773-799; C.F. LANE:"Reduction of Organic Compounds with Diborane" * page 774, column 2, paragraph 2, page 776, column 2, paragraph 4, page 790, column 1, paragraph 2 * | 1 | C 07 D 209/00<br>C 07 D 491/00 |
| A | M. HUDLICKY:"REDUCTIONS IN ORGANIC CHEMISTRY" 1984, Ellis Horwood Ltd., Chichester, W. Sussex * page 155, paragraph 3 * | 1 | |

The present search report has been drawn up for all claims

| Place of search | Date of completion of the search | Examiner |
|---|---|---|
| BERLIN | 14-07-1989 | KYRIAKAKOU G |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document